(19) Europäisches Patentamt
European Patent Office
Office européen des brevets

(11) Veröffentlichungsnummer: **0 157 360 B1**

(12) # EUROPÄISCHE PATENTSCHRIFT

(45) Veröffentlichungstag der Patentschrift: **14.08.91**

(51) Int. Cl.5: **C07D 275/04**, C07D 231/56, G01N 33/52, C12Q 1/34

(21) Anmeldenummer: **85103667.3**

(22) Anmeldetag: **27.03.85**

(54) **Chromogene Aminosäure-und Peptidester, Verfahren zu deren Herstellung, Verwendung dieser Verbindungen in Analysenverfahren sowie Mittel zum Nachweis esterolytischer und/oder proteolytischer Enzyme.**

(30) Priorität: **06.04.84 DE 3413077**

(43) Veröffentlichungstag der Anmeldung:
**09.10.85 Patentblatt 85/41**

(45) Bekanntmachung des Hinweises auf die Patenterteilung:
**14.08.91 Patentblatt 91/33**

(84) Benannte Vertragsstaaten:
**DE FR GB IT NL SE**

(56) Entgegenhaltungen:
**EP-A- 0 039 880**
**US-A- 3 929 815**

(73) Patentinhaber: **MILES INC.**
**1127 Myrtle Street**
**Elkhart Indiana 46514(US)**

(72) Erfinder: **Hugl, Herbert, Dr.**
**Gemarkenweg 9**
**W-5060 Bergisch Gladbach 2(DE)**
Erfinder: **Schnabel, Eugen, Dr.**
**Schimmelweg 6**
**W-5600 Wuppertal 11(DE)**

(74) Vertreter: **Dänner, Klaus, Dr. et al**
**c/o Bayer AG Konzernverwaltung RP Patentabteilung**
**W-5090 Leverkusen 1 Bayerwerk(DE)**

**EP 0 157 360 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue chromogene Aminosäure- und Peptidester von Hydroxybenz-(iso)thiazolen und Hydroxybenzpyrazolen, Verfahren zu deren Herstellung sowie die Verwendung der neuen Ester als Substrate für den analytischen Nachweis von esterolytischen und/oder proteolytischen Enzymen z.B. in Körperflüssigkeiten, wobei die Ester in geeigneter Weise in Testmitteln, insbesondere Teststreifen, inkorporiert sind. Bevorzugt werden die neuen Ester für den Nachweis von Leukozyten, insbesondere im Urin, eingesetzt.

In der Diagnostik der Erkrankungen der Nieren und des Urogenitaltraktes hat der Nachweis von Leukozyten in Körperflüssigkeiten, insbesondere im Urin, große Bedeutung. Ursprünglich erfolgte dieser Nachweis durch Auszählen der Leukozyten im nicht zentrifugierten Harn oder im Harnsediment. Mit beiden Methoden können nur intakte Leukozyten erfaßt werden. Es ist jedoch bekannt, daß die Geschwindigkeit der Leukozyten-Lyse je nach Harnmilieu starken Schwankungen unterworfen ist; so beträgt z.B. in stark alkalischen Harnen die Leukozyten-Halbwertszeit nur 60 Minuten. Dies führt dazu, daß zu niedrige Leukozytenzahlen festgestellt werden. Von diesem Lyse-Fehler abgesehen, liefert die quantitative mikroskopische Bestimmung der Leukozyten im nicht zentrifugierten, homogenisierten Harn in der Zählkammer sehr genaue Werte. Trotzdem wird diese Methode in der Praxis nur selten angewandt, da sie mühevoll und zeitraubend ist und geschultes Personal voraussetzt.

Das in der medizinischen Praxis bevorzugte Verfahren für die Leukozytenbestimmungen im Harn war daher die sogenannte Gesichtsfeldmethode im Harnsediment. Hierzu mußte zunächst die Probe (Sediment) durch Zentrifugieren gewonnen werden. Dabei wurden jedoch auch andere Bestandteile des Harnes angereichert, die - wie z.B. Salze und Epithelzellen - die mikroskopische Auszählung der Leukozyten beträchtlich erschweren. Schwankender Sedimentgehalt, Inhomogenitäten des Sediments sowie unterschiedliche optische Ausstattung der Mikroskope führten zu relativ großen Fehlern (bis zu mehreren hundert Prozent) bei der Angabe der Leukozytenzahl.

Um diese Schwierigkeiten zu vermeiden, wurde bereits vielfach versucht, als Nachweisprinzip für Leukozyten in verschiedenen Körperflüssigkeiten enzymatische Reaktionen heranzuziehen, da die Leukozyten ein breitgefächertes Enzymspektrum besitzen.

So sind z.B. aus den deutschen Offenlegungsschriften 2 826 965 und 2 836 644 Mittel zum Nachweis von Leukozyten in Körperflüssigkeiten bekannt, bei denen die in Leukozyten vorhandene esterolytische und/oder proteolytische Aktivität für analytische Zwecke ausgenutzt wird. Als Substrate für die Leukozyten-Esterasen und/oder -Proteasen werden dabei Sulfonphthaleinester bzw. Azo-Farbstoffester verwendet. Die bei der enzymatischen Umsetzung freigesetzten Farbstoffe werden dann nach bekannten Methoden bestimmt. Die in diesen Publikationen beschriebenen Mittel sind jedoch für praktische Zwecke noch zu unempfindlich, da sie bei niedrigen Leukozyten-Konzentrationen zu lange Reaktionszeiten aufweisen.

Verschiedene Methoden für den Nachweis von Proteasen und Esterasen sind auch aus der histo- und cytochemischen Enzymologie bekannt (vgl. beispielsweise A.G.E. Pearse, Histochemistry, Theoretical and Applied, 3. Ed., Churchill Livingstone, Edinburgh-London-New York 1968). Als Nachweis werden dabei im allgemeinen farblose oder schwach gefärbte Ester eingesetzt, die durch die Enzyme in eine farblose Säure und eine ebenfalls farblose Alkohol-(Phenol)-Komponente gespalten werden. Die Phenolkomponente wird dann in einer Folgereaktion zu farbigen Produkten umgesetzt, z.B. durch Kupplung mit Diazoniumsalzen oder durch Oxidation. F. Schmalzl und H. Braunsteiner beschreiben zum Beispiel in Klin. Wschr. 46, 642 (1968) einen spezifischen cytochemischen Leukozytenesterasenachweis mit Naphthol-AS-D-Chloracetat als Substrat und einem Diazoniumsalz, das mit dem freiwerdenden Naphthol eine farbige Azo-Verbindung bildet.

Für den schnellen und einfachen Nachweis von Leukozyten in Körperflüssigkeiten, wie z.B. im Harn, erwiesen sich Zweikomponenten-Systeme dieser Art jedoch als nicht geeignet, da sie viel zu unempfindlich sind: Proben mit 5000 Leukozyten/$\mu$l zeigen noch keine Reaktion.

In GB-A 1 128 371 und EP-A 12 957 wird die Verwendung von Indoxyl- und Thioindoxylestern als chromogenen Substraten für den Nachweis von hydrolytischen Enzymen in Körperflüssigkeiten beschrieben. Bei der enzymatischen Spaltung des Substrats entsteht freies Indoxyl, welches anschließend zum leicht nachweisbaren blauen Farbstoff Indigo oxidiert wird. Ein handelsüblicher Test auf Basis von EP-A 12 957 besteht aus einem Streifen Filterpapier, welches mit dem N-Tosyl-L-alanin-indoxylester imprägniert ist. Beim Eintauchen in eine Leukozyten enthaltende Urinprobe färbt sich der Teststreifen blau. Ein wesentlicher Nachteil dieses Produkts ist jedoch die lange Wartezeit (ca. 15 Minuten), bis die Endfärbung erreicht ist und der Test ausgewertet werden kann.

In EP-A 14 929 werden verschiedenartige Beschleuniger (Pyridinderivate; Imidazolderivate; Alkohole; Metallkomplexe) für die enzymatische Spaltungsreaktion beschrieben. Nachteilig bleiben jedoch die relativ

lange Zeit bis zur vollständigen Oxidation des Indoxyls und die geringe Empfindlichkeit des Tests (Nachweisgrenze: einige Tausend Leukozyten/µl). Gleiches trifft auch für die Verwendung der Ester von Leuko-Indoanilinen als Substrate für Leukozyten-Enzyme gemäß EP-A 34 323 zu.

EP-A 39 880 stellt eine Kombination der Substrate gemäß EP-A 12 957 bzw. 14 929 mit dem weiter oben diskutierten Nachweisprinzip der Kupplung mit Diazoniumsalzen dar. Es gelingt auf diese Weise zwar, die Erfassungsgrenzen für Leukozyten deutlich zu senken, jedoch wird die für die Praxisanwendung gewünschte Nachweisempfindlichkeit von 15 bis 20 Leukozyten/µl noch nicht erreicht.

In der US-PS 3,929,815 werden Alkylcarbamate von Benzoisothiazolen und deren Anwendung als Insektizide beschrieben.

Aufgabe der vorliegenden Erfindung war es daher, neue chromogene Substrate für esterspaltende Enzyme aufzufinden, die eine hohe Nachweisempfindlichkeit mit rascher Spaltung durch Leukozyten-Enzyme und rascher und intensiver Farbreaktion mit Diazoniumsalzen verbinden. Diese Aufgabe wird mit den erfindungsgemäßen Estern gelöst.

Gegenstand der Erfindung sind Verbindungen der allgemeinen Formel (I):

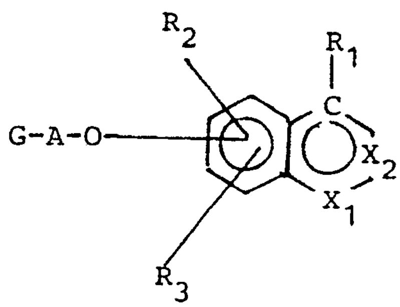

(I)

in welcher

- $X_1$ und $X_2$ gleich oder verschieden sind und Stickstoff oder Schwefel bedeuten, mit der Maßgabe, daß $X_1$ und $X_2$ nicht gleichzeitig für Schwefel stehen;
- $R_1$ für Wasserstoff oder eine gegebenenfalls verzweigte Alkylgruppe mit 1 bis 6 C-Atomen steht, die gegebenenfalls durch Halogen oder Hydroxy substituiert sein kann;
- $R_2$ und $R_3$ gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkylgruppen, $C_1$-$C_6$-Alkoxygruppen, Halogen, Cyano, Nitro, Trifluormethyl, $SO_3H$, $C_1$-$C_6$-Acylgruppen oder $C_1$-$C_6$-Dialkylaminogruppen substituiert sein können, oder $R_2$ und $R_3$ gemeinsam einen Benzolring, bilden, welcher seinerseits mit einem oder zwei Resten $R_2$ substituiert sein kann;

und G-A- einen Rest der allgemeinen Formel (IV)

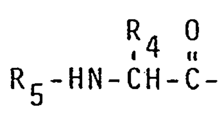

(IV)

dar, in welcher

- $R_4$ für Wasserstoff oder einen gegebenenfalls verzweigten Alkyl-, Cycloalkyl- oder Aralkylrest mit 1 bis 15 C-Atomen, steht, welcher gegebenenfalls durch eine oder zwei, Hydroxy-, Mercapto-, Carboxyl, Amino- oder Guanidinogruppe substituiert ist,

und

- $R_5$ Wasserstoff oder -CO-Alkyl, -CO-Aralkyl, -CO-Aryl, -$SO_2$-Alkyl oder -$SO_2$-Aryl darstellt, wobei Alkylreste geradkettige oder verzweigte Reste mit 1 bis 9 C-Atomen sind und die Arylreste 6 bis 12 C-Atome aufweisen und gegebenenfalls durch $C_1$-$C_4$-Alkoxygruppen oder Halogen substituiert sind.

Bevorzugt sind solche Verbindungen, worin $R_1$ für Wasserstoff steht. Besonders bevorzugt sind solche Verbindungen, worin $R_2$ und $R_3$, die gleich oder verschieden sind, für Wasserstoff, $C_1$-$C_2$-Alkyl, $C_1$-$C_2$-

Alkoxy, Halogen, $C_1$-$C_4$-Dialkylaminogruppen oder Benzolreste stehen. Ganz besonders bevorzugt sind solche Verbindungen, worin $R_2$ und $R_3$ für Wasserstoff stehen oder zusammen einen annellierten Benzolring bilden.

Gegenstand der Erfindung ist auch ein Verfahren zur Herstellung der erfindungsgemäßen Ester, welches dadurch gekennzeichnet ist, daß man ein Phenol der allgemeinen Formel (II):

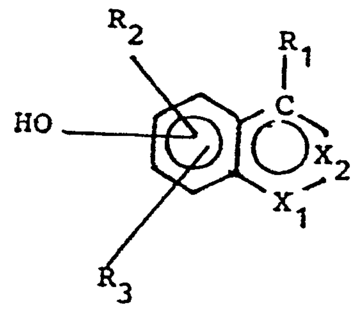
(II)

in welcher $X_1$, $X_2$, $R_1$, $R_2$ und $R_3$ die oben angegebene Bedeutung haben,
mit einem Rest der allgemeinen Formel III

G-A-OH (III)

in welcher G und A die oben angegebene Bedeutung haben, bzw. geeigneten reaktiven Derivaten davon nach in der Peptidchemie üblichen Methoden umsetzt.

Geeignete reaktive Derivate sind z.B. die Säurechloride und die bei der Peptidsynthese üblicherweise verwendeten Mischanhydride, z.B. mit Chlorameisensäureethylester, oder Aktivester wie z. B. Pentachlorphenylester oder N-Hydroxybenztriazolester.

Gegenstand der Erfindung ist weiterhin ein Mittel zum Nachweis esterolytischer und/oder proteolytischer Enzyme, enthaltend

(a) ein chromogenes Enzymsubstrat,
(b) ein Diazoniumsalz, gegebenenfalls
(c) einen Puffer sowie gegebenenfalls
(d) ein Trägermaterial und/oder übliche Zusatzstoffe,

dadurch gekennzeichnet, daß das chromogene Enzymsubstrat eine erfindungsgemäße Verbindung ist.

Gegenstand der Erfindung ist schließlich auch ein Verfahren zum Nachweis von esterolytischen und/oder proteolytischen Enzymen in flüssigen Proben, insbesondere Körperflüssigkeiten, welches dadurch gekennzeichnet ist, daß man die Probe mit den erfindungsgemäßen Mittel in Kontakt bringt und die auftretende Farbreaktion bestimmt.

Erfindungsgemäß bevorzugte Verbindungen der allgemeinen Formel (I) sind solche, bei denen $X_1$ für Schwefel oder Stickstoff und $X_2$ für Stickstoff stehen. Besonders bevorzugt stehen $X_1$ für Schwefel und $X_2$ für Stickstoff.

Bevorzugt sind weiterhin solche Verbindungen in welchen

$R_1$ für Wasserstoff

$R_2$ und $R_3$, die gleich oder verschieden sind, für Wasserstoff, $C_1$-$C_2$-Alkyl, $C_1$-$C_2$-Alkoxy, Halogen, $C_1$-$C_4$-Dialkylaminogruppen oder Benzolreste stehen.

Besonders bevorzugt stehen $R_2$ und $R_3$ für Wasserstoff.

Bevorzugt steht bei den Verbindungen der allgemeinen Formel (I) der Rest G-A-O- in 5-Position.

G-A- stellt einen Rest der allgemeinen Formel (IV)

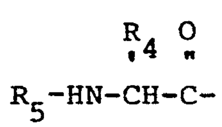
(IV)

dar, in welcher

$R_4$ für Wasserstoff oder einen gegebenenfalls verzweigten Alkyl-, Cycloalkyl-oder Aralkylrest mit 1 bis 15 C-Atomen, bevorzugt 1 bis 9 C-Atomen, steht, welcher gegebenenfalls durch eine oder

zwei, insbesondere eine, Hydroxy-, Mercapto-, Carboxyl, Amino- oder Guanidinogruppe substituiert ist, und

$R_5$ Wasserstoff oder vorzugsweise -CO-Alkyl, -CO-Aralkyl, -CO-Aryl, $-SO_2$-Alkyl oder $-SO_2$-Aryl darstellt, wobei Alkylreste geradkettige oder verzweigte Reste mit 1 bis 9 C-Atomen, bevorzugt 1 bis 6 C-Atomen, sind und die Arylreste 6 bis 12 C-Atome, vorzugsweise 6 C-Atome, aufweisen und gegebenenfalls durch $C_1$- bis $C_4$-Alkoxygruppen oder Halogen substituiert sind.

Besonders bevorzugt steht G-A- für einen mit einer üblichen Stickstoffschutzgruppe versehenen Rest einer natürlichen Aminosäure oder eines Peptids aus 2 bis 8 solcher Aminosäuren.

Die Aminosäurereste können dabei in ihrer L- oder D-Form oder auch in ihrer racemischen Form vorliegen. Besonders bevorzugt sind die Reste von Glycin, Alanin, Valin, Leucin, Isoleucin, Phenylalanin und Tyrosin, wobei jeweils die L-Form besonders bevorzugt ist. Gegebenenfalls vorhandene freie Hydroxygruppen können acyliert, vorzugsweise acetyliert, sein.

Unter einem Peptidrest in der Definition von A sind z.B. Di-, Tri-, Tetra- und Pentapeptide, vorzugsweise Di- und Tripeptide, zu verstehen, wobei als Aminosäure-Komponenten vorzugsweise die oben erwähnten Aminosäuren in Betracht kommen.

Als in der Peptidchemie übliche Stickstoffschutzgruppe in der Definition von G seien z.B. die an sich bekannten Alkyl- und Aralkyloxycarbonyl-, Alkyl- und Aralkyloxythiocarbonyl-, Sulfonyl-, Sulfenyl-, Vinyloxycarbonyl-, Cyclohexenyloxycarbonyl-, Phosphoryl- oder Carbamoyl-Gruppen genannt.

Die Phenole der allgemeinen Formel (II) sind an sich bekannt oder können nach bekannten Verfahren hergestellt werden.

Herstellungsverfahren für die Phenole der Formel (II) werden beispielsweise in folgenden Literaturstellen angegeben: Franke et.al., Arzneimittel-Forschung, 30 (11), 1831-1838; DE-A 2704 793; Davies, Soc. 1955, 2412; Clarke et.al. Condensed Isothiazoles, Part 7, J. Chem. Res., Synop., (6) 197 (1980), Hydroxyindazole in The Chemistry of Heterocyclic Compounds, Bd. 22, S. 336-340, Edited by R.H. Wiley.

Aus den Phenolen (II) und den Aminosäuren bzw. Peptiden der allgemeinen Formel (III) bzw. reaktiven Derivaten hiervon (insbesondere den Säurehalogeniden oder aktivierten Estern) lassen sich nach aus der Peptidchemie an sich bekannten Synthesemethoden die erfindungsgemäßen Verbindungen herstellen. Verfahren dieser Art werden beispielsweise in folgenden Publikationen (und den dort zitierten Literaturstellen) beschrieben: Janoff et al., Proc. Soc. Exper. Biol. Med. 136, 1045-1049 (1971); Sweetman et al., J. Hist. Soc., 22, 327-339; Jakubke et.al., Chem. Ber. 100, 2267-2372 (1967) sowie insbesondere Houben-Weyl, Methoden der organischen Chemie, Bd. XV/1 und XV/2.

Die erfindungsgemäßen Mittel zum Nachweis esterolytischer und/oder proteolytischer Enzyme enthalten neben den erfindungsgemäßen chromogenen Substraten ein Diazoniumsalz der allgemeinen Formel (V)

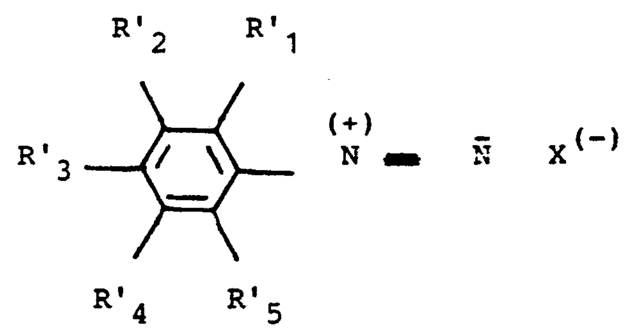

(V)

in der

$R'_1$ eine niedere Alkyl-, eine niedere Alkoxy-, eine niedere Alkylmercapto-, eine Hydroxy-, Nitro-, Cyano-, Trifluormethyl-, $C_1$-$C_8$-Alkylsulfonamido-, Arylsulfonamido-, $C_1$-$C_8$-Alkylsulfon-, Arylsulfon-, Sulfonsäure-, Carbonsäure-, eine N-Morpholino-, eine N-Thiomorpholino-, eine N-Pyrrolidino-, eine gegebenenfalls N'-alkylierte N-Piperazino-, eine N-Piperidino-Gruppe, Halogen oder Wasserstoff,

$R'_3$ eine niedere Alkyl-, eine niedere Alkoxy-, eine Aryloxy-, eine niedere Alkylmercapto-, Alkylamino-, Dialkylamino-, eine Hydroxy-, Nitro-, Cyano-, $C_1$-$C_8$-Alkylsulfonamido-, Arylsulfonamido-, $C_1$-$C_8$-Alkylsulfon-, Arylsulfon-, Sulfonsäure-, Carbonsäure-, eine N-Morpholino-, N-Thio-morpholino-, N-Pyrrolidino-, eine gegebenenfalls N'-alkylierte N-Piperazino-, N-Piperidino-, Phenylamino-, eine gegebenenfalls mit einem niederen Alkyl- oder niederen Alkoxy-Rest substituierte Phenyl-Gruppe, Halogen oder Wasser-

stoff,

$R'_2$, $R'_4$, $R'_5$, die gleich oder verschieden sein können, jeweils eine niedere Alkyl-, eine niedere Alkoxy-, Nitro-, $C_1$-$C_8$-Alkylsulfonamido-, Arylsulfonamido, $C_1$-$C_8$-Alkylsulfon-, Arylsulfon-, Sulfonsäure-, Carbonsäure-, eine niedere Alkylmercapto-Gruppe, Halogen oder Wasserstoff und

X ein stabilisierendes Anion

bedeuten, wobei jeweils 2 benachbarte Reste $R'_1$ bis $R'_5$ zu einem gegebenenfalls durch Halogen, eine $C_1$-$C_6$-Alkyl-, eine $C_1$-$C_6$-Alkoxy-, eine Nitro-, Sulfonsäure- oder Carbonsäuregruppe substituierten Benzolring ringgeschlossen sein können, so daß ein Diazoniumnsalz der Naphthalinreihe entsteht.

Vorzugsweise stehen in der allgemeinen Formel (V) die Reste

$R'_1$ für $C_1$- bis $C_4$-Alkyl, $C_1$-$C_4$-Alkoxy, Hydroxy, Nitro, Halogen oder Wasserstoff;

$R'_3$ für eine $C_1$- bis $C_4$-Alkyl-, $C_1$-$C_4$-Alkoxy-, Aryloxy-, $C_1$-$C_4$-Alkylamino-, $C_1$-$C_4$-Dialkylamino-, Nitro-, $C_1$-$C_4$-Alkylsulfonamido-, Arylsulfonamido-, $C_1$-$C_4$-Alkylsulfon-, Arylsulfon-, N-Morpholino-, N-Pyrrolidino-, Phenylamino-, oder Sulfonsäuregruppe oder Wasserstoff; und

$R'_2$, $R'_4$, $R'_5$, die gleich oder verschieden sein können, für $C_1$- bis $C_4$-Alkyl-, $C_1$- bis $C_4$-Alkoxy-, $C_1$- bis $C_4$-Alkylamino-,$C_1$-bis $C_4$-Dialkylamino-, Nitro-, $C_1$- bis $C_4$-Alkylsulfonamido-, Arylsulfonamido-, oder Sulfonsäuregruppen, Halogen oder Wasserstoff.

Jeweils zwei benachbare Reste $R'_1$ bis $R'_5$ können dabei zu einem gegebenenfalls durch Halogen,eine $C_1$- bis $C_4$-Alkyl-, $C_1$- bis $C_4$-Alkoxy-, Nitro- oder Sulfonsäuregruppe substituierten Benzolring ringgeschlossen sein.

Im Rahmen der Formel (V) steht Aryl jeweils für einen gegebenenfalls durch Halogen, eine $C_1$- $C_4$-Alkyl- oder $C_1$-$C_4$-Alkoxygruppe substituierten aromatischen Rest mit 6 bis 12 C-Atomen, vorzugsweise 6 C-Atomen.

Die Diazoniumsalze der allgemeinen Formel (V) sind ebenfalls an sich bekannt oder können nach bekannten Verfahren hergestellt werden (siehe Houben-Weyl, Methoden der organischen Chemie, Bd. X/3).

Vorzugsweise enthalten die erfindungsgemäßen Mittel Substanzen, welche die Spaltung des chromogenen Substrats (I) durch die nachzuweisenden Enzyme beschleunigen. Als solche Beschleuniger kommen z.B. die in EP-A 14 929 beschriebenen Verbindungen in Betracht, wobei aliphatische Alkohole mit 8 bis 25 C-Atomen, bevorzugt 10 - 20 C-Atomen, die gegebenenfalls ungesättigt sein können, bevorzugt sind. Beispiele solcher bevorzugten Beschleuniger sind n-Decanol, n-Dodecanol, und insbesondere n-Undecanol. Besonders bevorzugte Beschleuniger sind basische Aminosäuren enthaltende Homo- oder Copolyaminosäuren [E. Katchalski in: Advances of Protein Chemistry 13, 243-493 (1958); C.B. Anfinsen, M.L. Anson J.T. Edsall und K. Bailey (Hrsg.); Academic Press. Inc. Publishers, New York, N.Y.]sowie sequentielle Polyaminsäuren. Als basische Aminosäuren kommen solche Aminosäuren in Frage, die in den Seitenketten Amino- oder Guanidinogruppen tragen. Es sind dies insbesondere Lysin und Ornithin sowie Arginin, aber auch unnatürliche basische Aminosäuren wie beispielsweise Diaminobuttersäure, Diaminopropionsäure oder Diaminopimelinsäure. In den Polyaminosäuren können die konstituierenden Aminosäuren racemisch oder optisch aktiv in der D- oder L-Form vorliegen. Die Molekulargewichte (Zahlenmittel) der Polyaminosäuren betragen 1000 - 2000000 und liegen vorzugsweise zwischen 5000 und 500 000. Die Gehalte an basischen Aminosäuren können zwischen 5 und 100 Molprozent betragen, vorzugsweise zwischen 20 und 100 Molprozent.

Die Beschleuniger werden bei der Herstellung der unten beschriebenen Testvorrichtungen vorzugsweise in Mengen von 0,5 bis 10 Gew.-%, vorzugsweise 1 - 5 Gew.-% der Imprägnierlösung eingesetzt.

Vorzugsweise enthalten die erfindungsgemäßen Mittel zum Nachweis proteolytischer Enzyme und insbesondere der Leukozyten-Enzyme ein geeignetes Puffersystem. Hierfür kommen z.B. Phosphat-, Borat-, Carbonat/Hydrogencarbonat-, Carbonat-, Barbiturat-, Tris-(hydroxymethyl)-aminomethan-( = Tris), 2-Amino-2-methyl-propandiol-1,3- ( = Amediol)- oder Aminosäure-Puffer in Frage, wobei in der Regel pH-Wert und Kapazität so gewählt werden, daß sich in der Meßlösung bzw. auf dem Teststreifen ein pH-Wert von 6 - 10, vorzugsweise von 7 - 9, einstellt.

Die erfindungsgemäßen Mittel können auch an sich bekannte Detergentien enthalten, da hierdurch eine homogenere Farbverteilung und eine intensivere Färbung erzielt werden können. In Frage kommen sowohl kationaktive als auch anionaktive Detergentien aber auch amphotere und nichtionogene Detergentien. Als Beispiele hierfür seien Benzyl-dimethyl-tetradecylammoniumchlorid, Na-Dodecylsulfat, Zephirol, Polyvinylpyrrolidon, die oben als Aktivatoren genannten Polyaminosäuren sowie Heparinoid und Gemische dieser Verbindungen genannt.

Vorzugsweise sind bei den erfindungsgemäßen Mitteln die verschiedenen oben beschriebenen Reagenzien in einem inerten Trägermaterial der an sich bekannten Art inkorporiert, wobei als Trägermatrix

besonders bevorzugt poröse Materialien wie insbesondere Filterpapier, aber auch Kunststoffmembranen, Glasfasermatten (US-PS 3 846 247), poröse Keramikstreifen, Kunstfaservliese, schwammartige Materialien (US-PS 3 552 928), Filz, Textilien, Holz, Cellulose oder auch Silicagel in Frage kommen.

Die genannten Trägermaterialien werden zu diesem Zweck mit einer Lösung der oben beschriebenen Reagentien in einem geeigneten leicht entfernbaren Lösungsmittel, z.B. Wasser, Methanol, Ethanol, Aceton, DMF oder DMSO imprägniert. Vorzugsweise geschieht dies in zwei getrennten Schritten: Zunächst wird mit einer wäßrigen Lösung imprägniert, die den Puffer und andere wasserlösliche Zusatzstoffe enthält. Danach wird mit einer Lösung der chromogenen Enzym-Substrate der allgemeinen Formel (V) und Aktivatoren imprägniert. Die Imprägnierung kann jedoch auch in einer anderen Reihenfolge bzw. mit einer anderen Zusammensetzung der beiden Imprägnierlösungen durchgeführt werden. Vorzugsweise enthält die Imprägnierlösung oder die zu untersuchende Flüssigkeit die erfindungsgemäßen Verbindungen sowie das Diazoniumsalz in einer Konzentration von $10^{-4}$ mol/l bis $10^{-1}$ mol/l, insbesondere von $10^{-3}$ mol/l bis $10^{-2}$ mol/l.

Bei Verwendung von Filterpapier als Matrix können die fertigen Testpapiere als solche verwendet werden oder in an sich bekannter Weise an Griffen angeklebt oder vorzugsweise zwischen Kunststoffen und feinmaschigen Netzwerken z.B. gemäß DE-OS 2 118 455 eingesiegelt werden.

Zur Herstellung filmbeschichteter Teststreifen werden vorzugsweise sämtliche Reagenzien in die Lösung oder Dispersion einer filmbildenden Substanz, wie z.B. Polyvinylester oder Polyamid, eingetragen und homogen vermischt. Das Gemisch wird in dünner Schicht auf einen Kunststoffträger gestrichen und getrocknet. Die so hergestellten filmbeschichteten Teststreifen werden nach dem Trocknen geschnitten und können als solche verwendet oder in an sich bekannter Weise an Griffen angeklebt werden oder z.B. zwischen Kunststoffen und feinmaschigen Netzwerken gemäß DE-OS 2 118 455 eingesiegelt werden.

Ein erfindungsgemäßes diagnostisches Mittel zum Nachweis esterolytischer und/oder proteolytischer Enzyme, insbesondere der Leukozyten-Enzyme, in Form von Pulvermischungen oder Reagenztabletten läßt sich herstellen, indem die oben angeführten Bestandteile des Testmittels mit üblichen galenischen Zusatzstoffen versetzt und granuliert werden. Zusatzstoffe dieser Art sind z.B. Kohlenhydrate, wie z.B. Mono-, Oligo- oder Polysaccharide, oder Zuckeralkohole, wie z.B. Mannit, Sorbit oder Xylit, oder andere lösliche inerte Verbindungen, wie Polyethylenglykole oder Polyvinylpyrrolidon Die Pulvermischungen oder Reagenztabletten weisen z.B. ein Endgewicht von ungefähr 50 - 200 mg, vorzugsweise 50 - 80 mg, auf.

Zur Herstellung von Lyophilisaten im Gesamtgewicht von jeweils etwa 5 - 20 mg, vorzugsweise etwa 10 mg, wird eine Lösung gefriergetrocknet, die neben sämtlichen für den Test benötigten Reagenzien übliche Gerüstbildner, wie z.B. Polyvinylpyrrolidon, und evtl. weitere Füllstoffe, wie z.B. Mannit, Sorbit oder Xylit, enthält.

Ein erfindungsgemäßes diagnostisches Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z.B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Die so hergestellten diagnostischen Mittel ermöglichen es, nach Eintauchen in die zu untersuchende oder nach Zugabe zur betreffenden Körperflüssigkeit die Anwesenheit esterolytischer und/oder proteolytischer Enzyme, insbesondere der Leukozyten-Enzyme, rasch und einfach über eine Farbbildung nachzuweisen, die visuell oder photometrisch, z.B. remissions-photometrisch oder in der Küvette, gemessen werden kann. Da die Aktivität der Leukozyten-Enzyme, pro Zelle als eine im wesentlichen konstante Größe angesehen werden kann, läßt sich aus der Intensität der Farbbildung die Leukozytenkonzentration der untersuchten Körperflüssigkeit ermitteln. Dabei werden mit dem erfindungsgemäßen diagnostischen Mittel sowohl intakte als auch lysierte Leukozyten erfaßt, da die Aktivität der Leukozyten-Enzyme auch nach der Lyse der Leukozyten voll erhalten bleibt. Ein Lysefehler tritt folglich nicht auf.

Die nachfolgenden Beispiele dienen zur Erläuterung der vorliegenden Erfindung. Wenn nicht anders vermerkt, sind Mengenangaben als Gewichtsteile oder Gewichtsprozente zu verstehen.

Beispiel 1

Herstellung der N-Tosyl-L-alanylester:

Die Ester wurden jeweils z. B. durch Umsetzung von N-Tosyl-L-alanylchlorid mit den Phenolen in absolutem Methylethylketon oder absolutem Toluol in Gegenwart von gepulvertem Kaliumcarbonat hergestellt. Nach 6 bis 12-stündigem Rühren bei ca. 55° C waren zwischen 40 und 70 % des Phenols umgesetzt.

Das Molverhältnis Phenol: $K_2CO_3$: Säurechlorid betrug zumeist 1:1,5:1,5. Der pH-Wert lag während der gesamten Reaktionszeit um 7. Zur Aufarbeitung wurde das Kaliumcarbonat bei 50°C abfiltriert und danach das Lösungsmittel im Vakuum abdestilliert. Die Reinigung erfolgte über Säulenchromatographie mit Kieselgel (Laufmittel z. B. Petrolether: Aceton = ca. 9:1) und anschließendes Umkristallisieren.

p-Tosyl-L-alanin

Literatur: E. Fischer u. W. Lipschitz, B. 48, 362 (1915).

83,7 g (0,93 Mol) L-Alanin werden in 465 ml ca. 2N-Natronlauge gelöst. Die Lösung wird bei 70-72°C portionsweise mit 186 g (0,976 Mol) p-Toluolsulfochlorid in 20 Minuten versetzt. Während der Zugabe des Sulfochlorids wird die Reaktionsmischung durch einen automatischen Titrator mit ca. 2N-Natronlauge bei pH 10 gehalten; dabei werden 560 ml 2N-Natronlauge verbraucht. Wenn sich der pH der Reaktionsmischung nicht mehr ändert, kühlt man diese auf 15-5°C ab und stellt mit 37 %iger Salzsäure auf pH 3 ein. Das abgeschiedene Produkt wird abgesaugt, den feuchten Filterkuchen löst man aus 2350 ml Wasser um.
Ausbeute: 185,5 g (82 % der Theorie) L-p-Tosylalanin vom m.p. 132-135°C.

p-Tosyl-L-alanylchlorid

158,1 g (0,65 Mol) p-Tosyl-L-alanin werden in 350 ml Thionylchlorid bei 40°C gerührt, bis eine klare Lösung entstanden ist. Dann destilliert man das überschüssige Thionylchlorid in Wasserstrahlvakuum ab. Den Kolbenrückstand nimmt man in 300 ml destilliertem Toluol auf. Man erhält eine klare, schwach gelbliche Lösung, die in 900 ml gerührtes Waschbenzin eingegossen wird. Das Säurechlorid fällt aus. Es wird am nächsten Tage abgesaugt, mit Leichtbenzin gewaschen und in einem Vakuumexsiccator über Calciumchlorid/Kaliumhydroxyd getrocknet.
Ausbeute: 155 g (91 % der Theorie) an fast farblosen Kristallen vom m.p. 81-83°C.

5-[N-(Toluol-4''-sulfonyl)-L-alanyloxy]-1,2-benzisothiazol

3,02 g 5-Hydroxy-1,2-benzisothiazol werden in 150 ml absolutem Methylethylketon zusammen mit 3 g wasserfreiem $K_2CO_3$ unter Rühren auf 55°C erwärmt. Im Laufe von 6 Stunden gibt man portionsweise unter guten Rühren bei 55°C insgesamt 6 g N-Tosyl-L-alanylchlorid zu. Man rührt danach noch 2 Stunden bei 55°C nach. Nach dem Erkalten wird das $K_2CO_3$ abfiltriert, danach das Lösungsmittel abrotiert. Das Rohprodukt wird durch Hochdruckflüssigchromatographie an einer Kieselgelsäule gereinigt. Laufmittel : Hexan : Methylenchlorid : n-Butanol = 40 : 8 : 1. Detektion des gewünschten Produktes: UV bei 254 nm. Bei einer Aufgabenmenge von 3 mal 100 mg werden insgesamt 100 mg 5-[N-(Toluol-4''-sulfonyl)-L-alanyloxy]-1,2-benzisothiazol isoliert.

5-[N-(Toluol-4''-sulfonyl)-L-alanyloxy]-indazol

0,6 g 5-Hydroxy-indazol werden in 50 ml absolutem Methylenchlorid zusammen mit 1,2 g absolutem Pyridin bei 40°C vorgelegt. Im Laufe von 3 Stunden tropft man 1,5 g N-Tosyl-L-alanylchlorid, gelöst in 25 ml absoluten Methylenchlorid bei 40°C zu. Danach läßt man noch 2 Stunden bei 40°C nachrühren. Nach dem Erkalten wäscht man die Methylenchloridlösung 3mal mit je 100 ml 2%iger Citronensäurelösung. Danach wird die Methylenchloridphase über Natriumsulfat getrocknet und nach Abfiltrieren des Trockenmittels im Vakuum bei Raumtemperatur eingedampft. Der so erhaltene Rückstand wird säulenchromatographisch an Kieselgel, Laufmittel Petrolether : Aceton 6 : 4, gereinigt. Man erhält nach Abdampfen des Lösungsmittels der gewünschten Fraktion 0,4 g 5-[N-(Toluol-4''-sulfonyl)-L-alanyloxy]-indazol.

In analoger Weise erhält man durch Umsetzung der entsprechenden Hydroxybenzisothiazole und Hydroxyindazole der Formel (II) mit den jeweiligen N-geschützten Aminosäuren bzw. reaktiven Aminosäurederivaten die folgenden Substrate:
4-[N-(Toluol-4''-sulfonyl)-L-alanyloxy]-1,2-benzisothiazol
4-[N-(Toluol-4''-sulfonyl)-L-valyloxy]-1,2-benzisothiazol
5-[N-(t-Butyloxycarbonyl)-L-alanyloxy]-1,2-benzisothiazol
6-[N-Benzyloxycarbonyl-L-alanyloxy]-indazol
5-[N-Methylsulfonyl-L-lysyloxy]-indazol
4-[N-(4''-Methoxybenzolsulfonyl)-L-alanyloxy]-indazol
5-[N-Benzyloxycarbonyl-O-acetyl-L-tyrosyloxy]2,1-benzisothiazol
5-[N-Benzolsulfonyl-L-phenylalanyloxy]-1,2-benzisothiazol

6-[N-Benzyloxycarbonyl-L-valyloxy]-indazol

Beispiel 2

Filterpapier (z. B. Eaton and Dikeman 205) wird nacheinander mit den folgenden Lösungen imprägniert und dann bei 60 °C getrocknet.

Lösung 1
0,1 molarer Tris-(hydroxymethyl)-aminomethan-Puffer (pH8,4)
3 % Polyvinylpyrrolidon
2,5 % Poly-L-Arg
Lösungsmittel: Wasser

Lösung 2
$7{,}5 \times 10^{-3}$ mol/l 5-[N-(Toluol-4"-sulfonyl)-L-alanyloxy] -1,2-benzisothiazol
$1 \times 10^{-2}$ mol/l 2,4-Dimethoxy-benzoldiazoniumtetrafluoroborat
Lösungsmittel: wasserfreies Aceton
Man erhält ein schwach gelb gefärbtes Testpapier, das sich beim Eintauchen in leukozytenhaltige Urine rotbraun verfärbt.

Beispiel 3

Eine Tablette enthaltend
5 mg 5-[N-(Toluol-4"-sulfonyl)-L-alanyloxy]-1,2-benzisothiazol
5 mg 2,4-Dimethoxy-benzoldiazoniumtetrafluoroborat
4 mg Kaliumdihydrogenphosphat
80 mg Dinatriumhydrogenphosphat-dihydrat
6 mg Poly-L-Lys
110 mg Mannit
vermischt man mit 5 ml eines Leukozyten enthaltenden Urins. Die Urinprobe färbt sich rotbraun.

**Patentansprüche**

**1.** Verbindungen der allgemeinen Formel

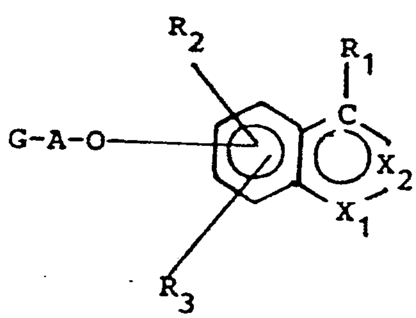

in welcher

| | |
|---|---|
| $X_1$ und $X_2$ | gleich oder verschieden sind und Stickstoff oder Schwefel bedeuten, mit der Maßgabe, daß $X_1$ und $X_2$ nicht gleichzeitig für Schwefel stehen; |
| $R_1$ | für Wasserstoff oder eine gegebenenfalls verzweigte Alkylgruppe mit 1 bis 6 C-Atomen steht, die gegebenenfalls durch Halogen oder Hydroxy substituiert sein kann; |
| $R_2$ und $R_3$ | gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkylgruppen, $C_1$-$C_6$-Alkoxygruppen, Halogen, Cyano, Nitro, Trifluormethyl, $SO_3H$, $C_1$-$C_6$-Acylgruppen oder $C_1$-$C_6$-Dialkylaminogruppen substituiert sein können, oder $R_2$ und $R_3$ gemeinsam einen Benzolring, bilden, |

welcher seinerseits mit einem oder zwei Resten $R_2$ substituiert sein kann;

und G-A- einen Rest der allgemeinen Formel (IV)

$$R_5\text{-HN-}\overset{R_4}{\underset{}{CH}}\text{-}\overset{O}{\overset{\|}{C}}\text{-} \qquad (IV)$$

dar, in welcher

$R_4$ für Wasserstoff oder einen gegebenenfalls verzweigten Alkyl-, Cycloalkyl- oder Aralkylrest mit 1 bis 15 C-Atomen, steht, welcher gegebenenfalls durch eine oder zwei, Hydroxy-, Mercapto-, Carboxyl, Amino- oder Guanidinogruppe substituiert ist, und

$R_5$ Wasserstoff oder -CO-Alkyl, -CO-Aralkyl, -CO-Aryl, $-SO_2$-Alkyl oder $-SO_2$-Aryl darstellt, wobei Alkylreste geradkettige oder verzweigte Reste mit 1 bis 9 C-Atomen sind und die Arylreste 6 bis 12 C-Atome aufweisen und gegebenenfalls durch $C_1$- bis $C_4$-Alkoxygruppen oder Halogen substituiert sind.

2. Verbindungen nach Anspruch 1, dadurch gekennzeichnet, daß $X_1$ für Schwefel und $X_2$ für Stickstoff stehen.

3. Verbindungen nach Anspruch 1 oder 2, dadurch gekennzeichnet, daß $R_1$ für Wasserstoff steht.

4. Verbindungen nach Ansprüchen 1 bis 3, dadurch gekennzeichnet, daß $R_2$ und $R_3$, die gleich oder verschieden sind, für Wasserstoff, $C_1$-$C_2$-Alkyl, $C_1$-$C_2$-Alkoxy, Halogen, $C_1$-$C_4$-Dialkylaminogruppen oder Benzolreste stehen.

5. Verbindungen nach Ansprüchen 1 bis 4, dadurch gekennzeichnet, daß $R_2$ und $R_3$ für Wasserstoff stehen oder zusammen einen annellierten Benzolring bilden.

6. Verbindungen nach Ansprüchen 1 bis 5, dadurch gekennzeichnet, daß die Gruppe G-A-O in 5-Position steht.

7. Verfahren zur Herstellung von Verbindungen der allgemeinen Formel

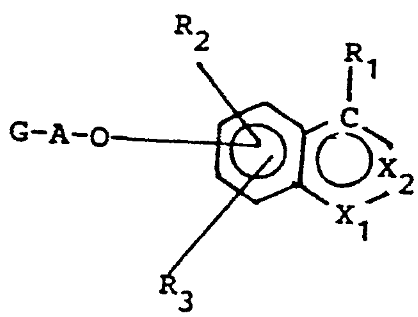

in welcher

$X_1$ und $X_2$ gleich oder verschieden sind und Stickstoff oder Schwefel bedeuten, mit der Maßgabe, daß $X_1$ und $X_2$ nicht gleichzeitig für Schwefel stehen;

$R_1$ für Wasserstoff oder eine gegebenenfalls verzweigte Alkylgruppe mit 1 bis 6 C-Atomen steht, die gegebenenfalls durch Halogen oder Hydroxy substituiert sein kann;

$R_2$ und $R_3$ gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkylgruppen, $C_1$-$C_6$-Alkoxygruppen, Halogen, Cyano, Nitro, Trifluormethyl, $SO_3H$, $C_1$-$C_6$-Acylgruppen oder $C_1$-$C_6$-Dialkylaminogruppen substituiert sein können, oder $R_2$ und $R_3$ gemeinsam einen Benzolring, bilden, welcher

seinerseits mit einem oder zwei Resten $R_2$ substituiert sein kann;

und G-A- einen Rest der allgemeinen Formel (IV)

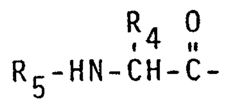

(IV)

dar, in welcher

$R_4$ für Wasserstoff oder einen gegebenenfalls verzweigten Alkyl-, Cycloalkyl- oder Aralkylrest mit 1 bis 15 C-Atomen, steht, welcher gegebenenfalls durch eine oder zwei, Hydroxy-, Mercapto-, Carboxyl, Amino- oder Guanidinogruppe substituiert ist, und

$R_5$ Wasserstoff oder -CO-Alkyl, -CO-Aralkyl, -CO-Aryl, -$SO_2$-Alkyl oder -$SO_2$-Aryl darstellt, wobei Alkylreste geradkettige oder verzweigte Reste mit 1 bis 9 C-Atomen sind und die Arylreste 6 bis 12 C-Atome aufweisen und gegebenenfalls durch $C_1$- bis $C_4$-Alkoxygruppen oder Halogen substituiert sind.

dadurch gekennzeichnet, daß man ein Phenol der allgemeinen Formel

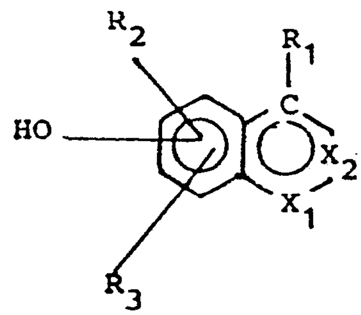

in welcher $X_1$, $X_2$, $R_1$, $R_2$ und $R_3$ die oben angegebene Bedeutung haben,

mit einem Rest der allgemeinen Formel

G-A-OH

in welcher G und A die oben angegebene Bedeutung haben, bzw. geeigneten reaktiven Derivaten davon nach in der Peptidchemie üblichen Methoden umsetzt.

**8.** Mittel zum Nachweis esterolytischer und/oder proteolytischer Enzyme, enthaltend
(a) ein chromogenes Enzymsubstrat,
(b) ein Diazoniumsalz, gegebenenfalls
(c) einen Puffer sowie gegebenenfalls
(d) ein Trägermaterial und/oder übliche Zusatzstoffe,
dadurch gekennzeichnet, daß das chromogene Enzymsubstrat eine Verbindung gemäß Anspruch 1 bis 6 ist.

**9.** Analyseverfahren zum Nachweis von esterolytischen und/oder proteolytischen Enzymen in flüssigen Proben, insbesondere Körperflüssigkeiten, dadurch gekennzeichnet, daß man die Probe mit einem Mittel gemäß Anspruch 8 in Kontakt bringt.

## Claims

**1.** Compounds of the general formula

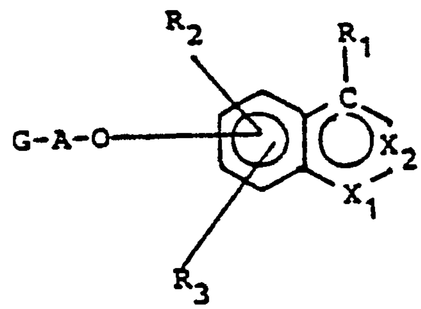

in which

$X_1$ and $X_2$ are identical or different and denote nitrogen or sulphur, with the proviso that $X_1$ and $X_2$ do not simultaneously represent sulphur;

$R_1$ represents hydrogen or an optionally branched alkyl group which has 1 to 6 C atoms and can optionally be substituted by halogen or hydroxyl;

$R_2$ and $R_3$ are identical or different and represent hydrogen, $C_1$-$C_6$-alkyl groups, $C_1$-$C_6$-alkoxy groups, $C_1$-$C_6$-acyl groups, halogen, trifluoromethyl, nitro, $SO_3H$, cyano, $C_1$-$C_8$-acylamino groups, $C_1$-$C_6$-dialkylamino groups or $C_6$-$C_{10}$-aryl groups, which can in turn be further substituted by $C_1$-$C_6$-alkyl groups, $C_1$-$C_6$-alkoxy groups, halogen, cyano, nitro, trifluoro-methyl, $SO_3H$, $C_1$-$C_6$-acyl groups or $C_1$-$C_6$-dialkylamino groups, or $R_2$ and $R_3$ together form a benzene ring, which can in turn be substituted by one or two radicals $R_2$;

and G-A- represents a radical of the general formula (IV)

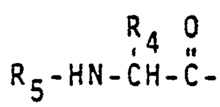

in which

$R_4$ represents hydrogen or an optionally branched alkyl, cycloalkyl or aralkyl radical which has 1 to 15 carbon atoms and is optionally substituted by one or two hydroxyl, mercapto, carboxyl, amino or guanidino group or groups, and

$R_5$ represents hydrogen or -CO-alkyl, -CO-aralkyl, -CO-aryl, -$SO_2$-alkyl or -$SO_2$-aryl, the alkyl radicals being straight-chain or branched radicals with 1 to 9 carbon atoms, and the aryl radicals containing 6 to 12 carbon atoms and optionally being substituted by $C_1$- to $C_4$-alkoxy groups or halogen.

**2.** Compounds according to Claim 1, characterised in that $X_1$ represents sulphur and $X_2$ represents nitrogen.

**3.** Compounds according to Claim 1 or 2, characterised in that $R_1$ represents hydrogen.

**4.** Compounds according to Claims 1 to 3, characterised in that $R_2$ and $R_3$, which are identical or different, represent hydrogen, $C_1$-$C_2$-alkyl, $C_1$-$C_2$-alkoxy, halogen, $C_1$-$C_4$-dialkylamino groups or benzene radicals.

**5.** Compounds according to Claims 1 to 4, characterised in that $R_2$ and $R_3$ represent hydrogen or together form a fused-on benzene ring.

**6.** Compounds according to Claims 1 to 5, characterised in that the G-A-O group is in the 5-position.

**7.** Process for the preparation of compounds of the general formula

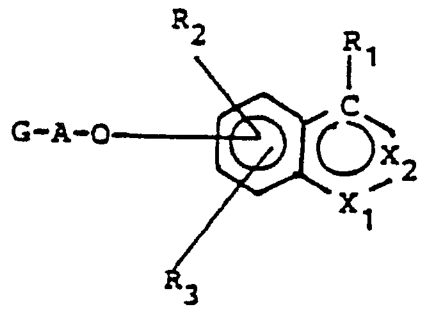

in which

$X_1$ and $X_2$ are identical or different and denote nitrogen or sulphur, with the proviso that $X_1$ and $X_2$ do not simultaneously represent sulphur;

$R_2$ represents hydrogen or an optionally branched alkyl group which has 1 to 6 C atoms and can optionally be substituted by halogen or hydroxyl;

$R_1$ and $R_3$ are identical or different and represent hydrogen, $C_1$-$C_6$-alkyl groups, $C_1$-$C_6$-alkoxy groups, $C_1$-$C_6$-acyl groups, halogen, trifluoromethyl, nitro, $SO_3H$, cyano, $C_1$-$C_8$-acylamino groups, $C_1$-$C_6$-dialkylamino groups or $C_6$-$C_{10}$-aryl groups, which can in turn be further substituted by $C_1$-$C_6$-alkyl groups, $C_1$-$C_6$-alkoxy groups, halogen, cyano, nitro, trifluoromethyl, $SO_3H$, $C_1$-$C_6$-acyl groups or $C_1$-$C_6$-dialkylamino groups, or $R_2$ and $R_3$ together form a benzene ring, which can in turn be substituted by one or two radicals $R_2$;

and G-A- represents a radical of the general formula (IV)

$$R_5\text{-HN-}\underset{}{\overset{R_4}{CH}}\text{-}\overset{O}{\overset{\|}{C}}\text{-}$$

in which

$R_4$ represents hydrogen or an optionally branched alkyl, cycloalkyl or aralkyl radical which has 1 to 15 carbon atoms and is optionally substituted by one or two hydroxyl, mercapto, carboxyl, amino or guanidino group or groups, and

$R_5$ represents hydrogen or -CO-alkyl, -CO-aralkyl, -CO-aryl, - $SO_2$-alkyl or -$SO_2$-aryl, the alkyl radicals being straight-chain or branched radicals with 1 to 9 carbon atoms, and the aryl radicals containing 6 to 12 carbon atoms and optionally being substituted by $C_1$- to $C_4$-alkoxy groups or halogen,

characterised in that a phenol of the general formula

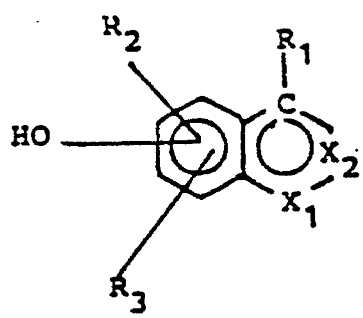

in which $X_1$, $X_2$, $R_1$, $R_2$ and $R_3$ have the above-mentioned meaning,

is reacted with a radical of the general formula

G-A-OH

in which G and A have the abovementioned meaning, or suitable reactive derivatives thereof, by methods customary in peptide chemistry.

**8.** Agents for the detection of esterolytic and/or proteolytic enzymes containing
(a) a chromogenic enzyme substrate,
(b) a diazonium salt, if appropriate
(c) a buffer, and if appropriate
(d) a carrier and/or customary additives,
characterised in that the chromogenic enzyme substrate is a compound according to Claim 1 to 6.

**9.** Analytical process for the detection of esterolytic and/or proteolytic enzymes in liquid samples, in particular body fluids, characterised in that the sample is brought into contact with an agent according to Claim 8.

**Revendications**

**1.** Composés de formule générale

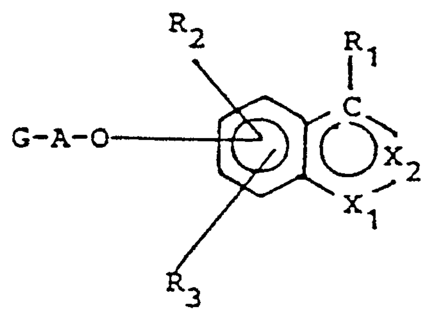

dans laquelle

| | |
|---|---|
| $X_1$ et $X_2$ | sont identiques ou différents et représentent l'azote ou le soufre, sous réserve que $X_1$ et $X_2$ ne représentent pas simultanément le soufre ; |
| $R_1$ | est l'hydrogène ou un groupe alkyle en $C_1$ à $C_6$ éventuellement ramifié, qui peut être substitué le cas échéant par un halogène ou un groupe hydroxy ; |
| $R_2$ et $R_3$ | sont identiques ou différents et représentent l'hydrogène, des groupes alkyle en $C_1$ à $C_6$, des groupes alkoxy en $C_1$ à $C_6$, des groupes acyle en $C_1$ à $C_6$, un halogène, un groupe trifluorométhyle, nitro, $SO_3H$, cyano, des groupes acylamino en $C_1$ à $C_8$, des groupes di(alkyle en $C_1$ à $C_6$)amino ou des groupes aryle en $C_6$ à $C_{10}$, qui peuvent être substitués quant à eux par des groupes alkyle en $C_1$ à $C_6$, des groupes alkoxy en $C_1$ à $C_6$, un halogène, un groupe cyano, nitro, trifluorométhyle, $SO_3H$, des groupes acyle en $C_1$ à $C_6$ ou des groupes di(alkyle en $C_1$ à $C_6$)amino, ou bien $R_2$ et $R_3$ forment conjointement un noyau benzénique, qui peut être substitué quant à lui avec un ou deux restes $R_2$ ; et |
| G-A- | représente un reste de formule générale (IV) |

$$R_5\text{-HN-}\overset{R_4}{\overset{|}{CH}}\text{-}\overset{O}{\overset{\|}{C}}\text{-} \qquad (IV)$$

dans laquelle

| | |
|---|---|
| $R_4$ | est l'hydrogène ou un reste alkyle éventuellement ramifié, cycloalkyle ou aralkyle ayant 1 à 15 atomes de carbone, qui porte, le cas échéant, un ou deux substituants hydroxy, |

mercapto, carboxyle, amino ou guanidino,
et

$R_5$ est l'hydrogène ou un groupe -CO-alkyle, -CO-aralkyle, -CO-aryle, $-SO_2$-alkyle ou $-SO_2$-aryle, les restes alkyle étant des restes à chaîne droite ou ramifiés ayant 1 à 9 atomes de carbone et les restes aryle présentant 6 à 12 atomes de carbone et étant substitués, le cas échéant, par des groupes alkoxy en $C_1$ à $C_4$ ou par un halogène.

2. Composés suivant la revendication 1, caractérisés en ce que $X_1$ représente le soufre et $X_2$ représente l'azote.

3. Composés suivant la revendication 1 ou 2, caractérisés en ce que $R_1$ représente l'hydrogène.

4. Composés suivant les revendications 1 à 3, caractérisés en ce que $R_2$ et $R_3$, qui sont identiques ou différents, représentent l'hydrogène, des groupes alkyle en $C_1$ ou $C_2$, alkoxy en $C_1$ ou $C_2$, un halogène, des groupes di(alkyle en $C_1$ à $C_4$)amino ou des restes benzéniques.

5. Composés suivant les revendications 1 à 4, caractérisés en ce que $R_2$ et $R_3$ représentent l'hydrogène ou forment conjointement un noyau benzénique condensé.

6. Composés suivant les revendications 1 à 5, caractérisés en ce que le groupe G-A-O est en position 5.

7. Procédé de préparation de composés de formule générale

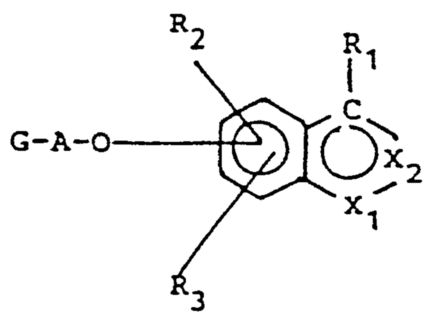

dans laquelle

$X_1$ et $X_2$ sont identiques ou différents et représentent l'azote ou le soufre, sous réserve que $X_1$ et $X_2$ ne représentent pas simultanément le soufre ;

$R_1$ est l'hydrogène ou un groupe alkyle en $C_1$ à $C_6$ éventuellement ramifié, qui peut être substitué le cas échéant par un halogène ou un groupe hydroxy ;

$R_2$ et $R_3$ sont identiques ou différents et représentent l'hydrogène, des groupes alkyle en $C_1$ à $C_6$, des groupes alkoxy en $C_1$ à $C_6$, des groupes acyle en $C_1$ à $C_6$, un halogène, un groupe trifluorométhyle, nitro, $SO_3H$, cyano, des groupes acylamino en $C_1$ à $C_8$, di-(alkyle en $C_1$ à $C_6$)amino ou des groupes aryle en $C_6$ à $C_{10}$, qui peuvent être substitués quant à eux par des groupes alkyle en $C_1$ à $C_6$, des groupes alkoxy en $C_1$ à $C_6$, un halogène, un groupe cyano, nitro, trifluorométhyle, $SO_3H$, des groupes acyle en $C_1$ à $C_6$ ou des groupes di(alkyle en $C_1$ à $C_6$)amino, ou bien $R_2$ et $R_3$ forment conjointement un noyau benzénique, qui peut être substitué quant à lui avec un ou deux restes $R_2$ ;
et

G-A- représente un reste de formule générale (IV)

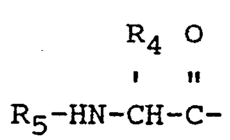

(IV)

dans laquelle

$R_4$ est l'hydrogène ou un reste alkyle éventuellement ramifié, cycloalkyle ou aralkyle ayant 1 à 15 atomes de carbone, qui porte, le cas échéant, un ou deux substituants hydroxy, mercapto, carboxyle, amino ou guanidino,
et
$R_5$ est l'hydrogène ou un groupe -CO-alkyle, -CO-aralkyle, -CO-aryle, $-SO_2$-alkyle ou $-SO_2$-aryle, les restes alkyle étant des restes à chaîne droite ou ramifiés ayant 1 à 9 atomes de carbone et les restes aryle présentant 6 à 12 atomes de carbone et étant substitués, le cas échéant, par des groupes alkoxy en $C_1$ à $C_4$ ou par un halogène,

caractérisé en ce qu'on fait réagir un phénol de formule générale

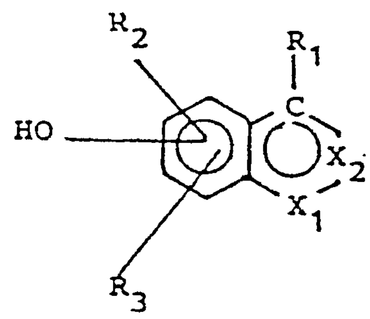

dans laquelle $X_1$, $X_2$, $R_1$, $R_2$ et $R_3$ ont la définition indiquée ci-dessus,
avec un reste de formule générale

G-A-OH

dans laquelle G et A ont la définition indiquée ci-dessus ou leurs dérivés réactifs appropriés, selon des opérations d'emploi courant dans la chimie des peptides.

**8.** Agent pour la détection d'enzymes estérolytiques et/ou protéolytiques, contenant
(a) un substrat enzymatique chromogène
(b) un sel de diazonium, le cas échéant
(c) un tampon ainsi que, le cas échéant
(d) un support et/ou un additif classique,
caractérisé en ce que le substrat chromogène d'enzyme est un composé suivant les revendications 1 à 6.

**9.** Méthode d'analyse pour la détection d'enzymes estérolytiques et/ou protéolytiques dans des échantillons liquides, notamment dans les liquides corporels, caractérisée en ce que l'échantillon est mis en contact avec un agent suivant la revendication 8.